# EUROPEAN PATENT SPECIFICATION

(11) **EP 3 266 404 B1**
(45) Date of publication and mention of the grant of the patent: **02.09.2020**
(21) Application number: 17179833.3
(22) Date of filing: 05.07.2017
(51) Int. Cl.: A61C 1/00, A61L 2/24, A61C 1/05, A61L 2/18, A61L 2/10

(54) **MEDICAL DEVICE AND MEDIUM CONDUIT SET**
MEDIZINISCHE VORRICHTUNG UND MEDIUMLEITUNGSSATZ
DISPOSITIF MÉDICAL ET ENSEMBLE DE CONDUIT POUR MÉDIUM

(30) Priority: 06.07.2016 JP 2016134292
(43) Date of publication of application: 10.01.2018
(73) Proprietor: J. MORITA MANUFACTURING CORPORATION, Fushimi-ku, Kyoto-shi, Kyoto 6128533 (JP)
(72) Inventor: TANAKA, Noriyuki, Kyoto, 612-8533 (JP); FURUTA, Yoshikazu, Kyoto, 612-8533 (JP); OZAWA, Keigo, Kyoto, 612-8533 (JP)
(74) Representative: Müller Hoffmann & Partner

(56) References cited:
- WO-A2-01/54611
- DE-U1- 8 907 473
- US-A- 4 545 956
- US-A1- 2002 134 736
- US-B1- 7 056 472

## Description

### Field of the Invention

The present invention relates to a medical device and a medium conduit set.

### Description of the Background Art

A dental treatment device representing one example of a medical device includes a treatment main unit and a treatment handpiece. While the treatment handpiece is used, water is supplied from the treatment main unit to the treatment handpiece via a water feed tube. After the treatment handpiece is used, water remains in the water feed tube. If water remains in the water feed tube for a long period of time for holidays or the like, germs may grow in the water feed tube. Therefore, before the treatment handpiece is used, water which remains in the water feed tube should be drained and fresh water should be fed to the water feed tube for cleaning the water feed tube.

For example, Japanese Patent No. 4348075 describes a dental treatment device for feeding a treatment liquid to which a degerminating agent has been added into a feed line (a water feed line) to thereby clean the feed line.

Since the dental treatment device described in the publication cleans the feed tube with a medicinal solution, time and efforts are required for adjusting the medicinal solution or the like. Influence by the medicinal solution onto human bodies should also be avoided.

### SUMMARY OF THE INVENTION

The invention pertains to a medical device as defined by the appended claims. The present invention was made in view of the problems above, and an object is to provide a medical device and a medium conduit set which can be simplified in cleaning of a supply tube.

A medical device according to the present invention includes a treatment main unit, a flexible medium conduit connected to the treatment main unit, and a treatment handpiece connected to the medium conduit and connected to the treatment main unit with the medium conduit being interposed. The medium conduit includes an outer tube, a supply tube arranged in the outer tube, through which water is supplied from the treatment main unit to the treatment handpiece, and an emission tube arranged in the outer tube and connected to the supply tube, through which the water is emitted from the medium conduit to the treatment main unit. The medical device according to the present invention further includes a valve configured to allow the water to flow either to the treatment handpiece from the supply tube or to the treatment main unit via the emission tube.

According to the medical device in the present invention, the valve is configured to allow water to flow either to the treatment handpiece from the supply tube or to the treatment main unit via the emission tube. Therefore, water can be fed from the supply tube to the emission tube. Therefore, since the supply tube can be cleaned with water, cleaning of the supply tube can be simplified.

The foregoing and other objects, features, aspects and advantages of the present invention will become more apparent from the following detailed description of the present invention when taken in conjunction with the accompanying drawings.

### BRIEF DESCRIPTION OF THE DRAWINGS

Fig. 1 is a perspective view schematically showing a configuration of a medical device in one embodiment of the present invention.
Fig. 2 is a perspective view schematically showing a tray table, a medium conduit, and a treatment handpiece shown in Fig. 1.
Fig. 3 is a perspective cross-sectional view along the line III-III in Fig. 2.
Fig. 4 is a diagram schematically showing tubing in the medical device in one embodiment of the present invention.
Fig. 5 is a control block diagram of the medical device in one embodiment of the present invention.
Fig. 6 is a timing chart of a method of cleaning the medical device in one embodiment of the present invention.
Fig. 7 is a flowchart of the method of cleaning the medical device in one embodiment of the present invention.
Fig. 8 is a flowchart of another method of cleaning the medical device in one embodiment of the present invention.
Fig. 9 is a diagram schematically showing tubing in a first modification of the medical device in one embodiment of the present invention.
Fig. 10 is a diagram schematically showing tubing in a second modification of the medical device in one embodiment of the present invention.
Fig. 11 is a diagram schematically showing tubing in a third modification of the medical device in one embodiment of the present invention.
Fig. 12 is an assembly cross-sectional view of a tip end portion of the medium conduit for attachment to, removal from, and engagement with the treatment handpiece shown in Fig. 2.

### DESCRIPTION OF THE PREFERRED EMBODIMENTS

An embodiment of the present invention will be described below with reference to the drawings.

A configuration of a medical device in one embodiment of the present invention will initially be described. In the present embodiment, a dental treatment device will be described by way of example of a medical device.

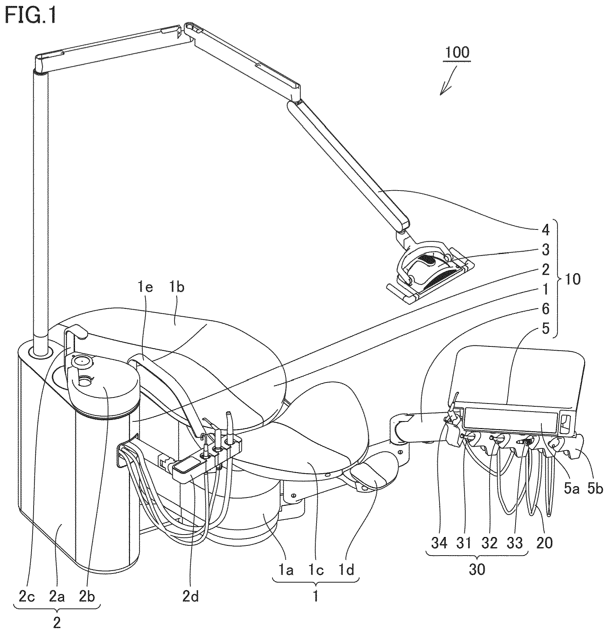

Referring to Fig. 1, a medical device 100 according to the present embodiment includes a treatment main unit 10, a medium conduit 20, and a treatment handpiece 30. Medium conduit 20 and treatment handpiece 30 is defined as a medium conduit set. The medium conduit set is configured to removably be connected to treatment main unit 10.

Treatment main unit 10 includes a treatment table 1, a spittoon 2, a lighting device 3, a lighting device arm 4, a tray table 5, and a tray table arm 6. Treatment table 1 is configured such that a patient can receive treatment while the patient is seated or lies on the patient's back. Treatment table 1 includes a base 1a, a seat 1b, a backrest 1c, a headrest 1d, and an armrest 1e. Seat 1b is set on base 1a so as to be raised and lowered. Backrest 1c is attached to one end of seat 1b as being reclinable. Headrest 1d is attached to an upper end of backrest 1c so as to be tilted. Arm rest 1e is set laterally to seat board 1b.

Spittoon 2 is set laterally to treatment table 1. Spittoon 2 includes a body portion 2a, a basin 2b, and a cup water faucet (a cup water feed portion) 2c. Basin 2b is set at an upper end of body portion 2a. Cup water faucet 2c is set over basin 2b. Lighting device 3 is connected to spittoon 2 with lighting device arm 4 being interposed. For example, an shadowless lamp is employed as lighting device 3.

Tray table 5 is connected to base 1a with tray table arm 6 being interposed. Tray table 5 is used for placing or holding treatment instruments, medications, and treatment handpieces (instruments) 30. Tray table 5 includes an operation setting portion 5a for setting an operation of treatment table 1 and treatment handpiece 30.

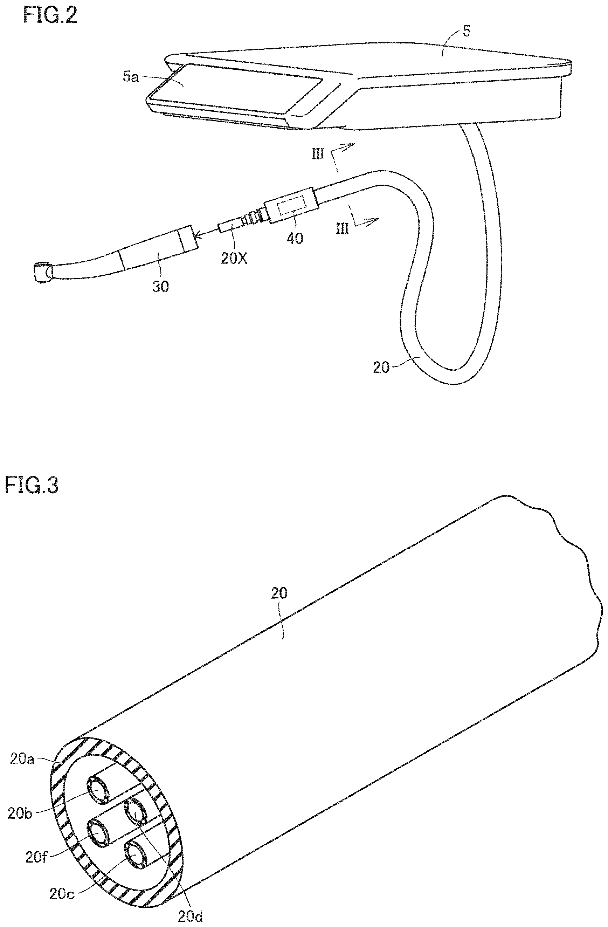

Referring to Figs. 1 and 2, medium conduit 20 is configured to supply functional media such as electric power, air, or water from treatment main unit 10 to treatment handpiece 30. Fig. 2 shows one medium conduit 20 and one treatment handpiece 30 for the sake of ease of view. Fig. 2 shows treatment handpiece 30 as being removed from medium conduit 20 for the sake of convenience of illustration.

Medium conduit 20 is connected to treatment main unit 10 and treatment handpiece 30. Specifically, spittoon 2 and treatment handpiece 30 are connected to an assistant-side handpiece holder 2d which is a part of a plurality of medium conduits 20, and a doctor-side handpiece holder 5b which is another part of the plurality of medium conduits 20 is arranged under tray table 5 and connects tray table 5 and treatment handpiece 30 to each other. Medium conduit 20 is flexible. Medium conduit 20 includes at a tip end, an engagement portion 20X for engagement with treatment handpiece 30. As shown with an arrow in Fig. 2, engagement portion 20X is inserted into treatment handpiece 30 from one end of treatment handpiece 30. Medium conduit 20 is thus removably connected to treatment handpiece 30.

An air turbine handpiece will be described by way of example with reference to Figs. 2 and 3. Medium conduit 20 includes an outer tube 20a, a supply tube 20b through which water is supplied, an emission tube 20c through which water is emitted, an air feed tube 20d through which driving air is supplied, and an exhaust tube 20f through which driving air is exhausted. Outer tube 20a is tubular. Supply tube 20b, air feed tube 20d, exhaust tube 20f, and emission tube 20c are arranged in outer tube 20a. Supply tube 20b, air feed tube 20d, exhaust tube 20f, and emission tube 20c are arranged as being aligned in outer tube 20a. Outer tube 20a is made, for example, of silicone. Each of supply tube 20b, air feed tube 20d, exhaust tube 20f, and emission tube 20c has, for example, a two-layered structure; an inner layer of the two-layered structure is made of a fluorine resin and an outer layer thereof is made of urethane. Supply tube 20b is configured to supply water from treatment main unit 10 to treatment handpiece 30. Supply tube 20b serves as a water feed tube through which water is supplied from treatment main unit 10 to treatment handpiece 30. Emission tube 20c is configured to emit water from medium conduit 20 to treatment main unit 10. Emission tube 20c serves as a drain tube through which water is emitted from medium conduit 20 to treatment main unit 10. Air feed tube 20d is an air feed tube through which driving air is supplied to treatment handpiece 30. Though the air feed tube through which driving air is supplied or the exhaust tube through which driving air is exhausted are essential in the air turbine handpiece, it is not necessary to provide an exhaust tube in a micromotor handpiece, an ultrasonic handpiece, or the like.

Referring to Figs. 1 and 2, treatment handpiece 30 is a dental treatment instrument. Treatment handpiece 30 may include a plurality of handpieces. Treatment handpiece 30 is exemplified by a first air turbine handpiece (a high-speed handpiece) 31, a second air turbine handpiece (a high-speed handpiece) 32, a micromotor handpiece (a low-speed handpiece) 33, a three-way syringe 34, or the like. Treatment handpiece 30 is configured to be locked to doctor-side handpiece holder 5b provided in tray table 5 and assistant-side handpiece holder 2d provided in spittoon body portion 2a.

Referring to Figs. 1, 2, and 4, medical device 100 further includes a valve 40. Valve 40 is configured to allow water to flow either to treatment handpiece 30 from supply tube 20b through which water is supplied or to treatment main unit 10 via emission tube 20c through which water is emitted. Valve 40 may be arranged in medium conduit 20 or in treatment handpiece 30. Valve 40 may be arranged in at least any of medium conduit 20, treatment handpiece 30, and treatment main unit 10 as being separate.

In the present embodiment, valve 40 includes a supply on-off valve 40a, a supply tube on-off valve 40b, and an emission on-off valve 40c. An electromagnetic valve may be employed as each of supply on-off valve 40a, supply tube on-off valve 40b, and emission on-off valve 40c. Supply on-off valve 40a is arranged between a main water feed tube (medium supply portion) 51 connected to treatment handpiece 30 from a source of water (tap water) 50a in treatment main unit 10 and supply tube 20b through which water in medium conduit 20 is supplied. Supply tube on-off valve 40b is arranged between a connection portion of supply tube 20b for water supply to which emission tube 20c for emitting water in medium conduit 20 is connected and treatment handpiece 30. Supply tube on-off valve 40b is desirably provided in a portion of connection of medium conduit 20 to treatment handpiece 30. Emission on-off valve 40c is arranged between a drain portion (a medium emission portion) 140 and emission tube 20c through which water in medium conduit 20 is emitted. A portion of branch of supply tube 20b and emission tube 20c is provided immediately before an upstream side of supply tube on-off valve 40b arranged in medium conduit 20.

Medical device 100 is configured to allow water to flow from supply tube 20b through which water in medium conduit 20 is supplied via emission tube 20c through which water is emitted to treatment main unit 10 by opening supply on-off valve 40a, closing supply tube on-off valve 40b, and opening emission on-off valve 40c.

Medical device 100 is configured to allow water to flow from supply tube 20b through which water for at least any of the handpieces is supplied via emission tube 20c through which water is emitted to treatment main unit 10 by controlling opening and closing of supply on-off valve 40a and supply tube on-off valve 40b provided for each handpiece.

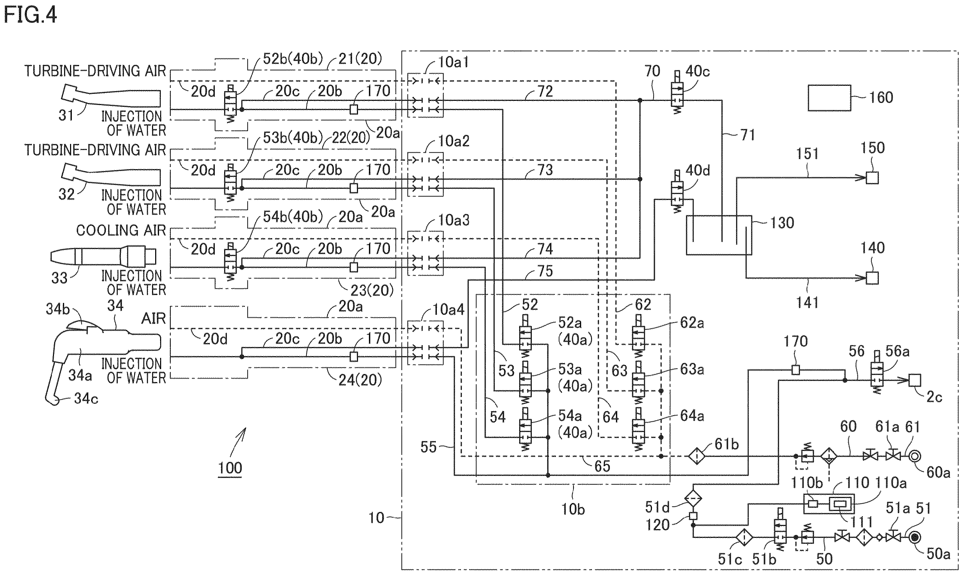

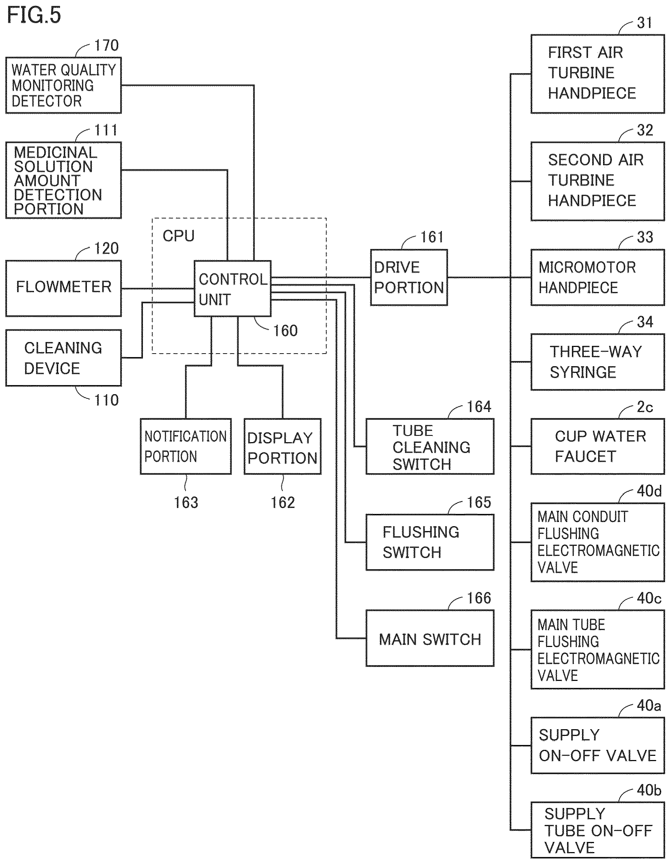

Tubing of medical device 100 according to the present embodiment will now be described with reference to Figs. 4 and 5. Fig. 4 does not show exhaust tube 20f of first air turbine handpiece 31 and second air turbine handpiece 32 for avoiding complication of the figure.

Treatment main unit 10 includes a tube 50 for water feed (corresponding to supply tube 20b), an air supply tube 60 (corresponding to air feed tube 20d), a drainage water tube 70 (corresponding to emission tube 20c), a cleaning device 110, a flowmeter 120, a cleaning tank 130, a drain portion 140, a vacuum tank 150, a control unit 160, and a water quality monitoring detector 170.

Tube 50 for water feed includes a main water feed tube 51, a first branch water feed tube 52 through which water is fed to first air turbine handpiece 31, a second branch water feed tube 53 through which water is fed to second air turbine handpiece 32, a third branch water feed tube 54 through which water is fed to micromotor handpiece 33, a fourth branch water feed tube 55 through which water is fed to three-way syringe 34, and a fifth branch water feed tube 56 through which water is fed to cup water faucet 2c. First branch water feed tube 52, second branch water feed tube 53, third branch water feed tube 54, fourth branch water feed tube 55, and fifth branch water feed tube 56 are branched from main water feed tube 51.

Main water feed tube 51 is connected to source of water (tap water) 50a for treatment. Main water feed tube 51 is provided with a manual source valve 51a, a water feed source electromagnetic valve 51b, a sterilization filter 51c, flowmeter 120, and a warmer tank 51d sequentially from the upstream side. Main water feed tube (medium supply portion) 51 is configured such that water is supplied to supply tube 20b of medium conduit 20.

Flowmeter 120 is attached to main water feed tube 51. Flowmeter 120 serves to measure a flow rate of water which flows through main water feed tube 51. Flowmeter 120 is arranged on a downstream side of a portion of connection between main water feed tube 51 and cleaning device 110.

Water quality monitoring detector 170 is attached to main water feed tube 51. Water quality monitoring detector 170 is a detector for monitoring quality of water which remains in at least any of treatment main unit 10 and supply tube 20b through which water is supplied. Therefore, water quality monitoring detector 170 may also be attached to emission tube 20c through which water in medium conduit 20 is emitted. Supply on-off valve 40a and supply tube on-off valve 40b may be configured to automatically be activated in response to an output from water quality monitoring detector 170.

A first branch drain tube 72 of first air turbine handpiece 31, a second branch drain tube 73 of second air turbine handpiece 32, and a third branch drain tube 74 of micromotor handpiece 33 merge to be drainage water tube 70 so that water is drained from first main drain tube 71 to cleaning tank 130 through a main tube flushing electromagnetic valve (emission on-off valve) 40c.

Water is drained to cleaning tank 130 through a second main drain tube 75 of three-way syringe 34 via a main conduit flushing electromagnetic valve 40d.

First branch water feed tube 52 is connected to first air turbine handpiece 31 with a main tube 21 (medium conduit 20) of first air turbine handpiece 31 being interposed. One end (a first end) of supply tube 20b of main tube 21 of first air turbine handpiece 31 is connected to first branch water feed tube 52 at a first main tube attachment and removal portion 10a1 of treatment main unit 10. The other end (a second end) of supply tube 20b of main tube 21 of first air turbine handpiece 31 is connected to first air turbine handpiece 31. First branch water feed tube 52 is provided with a first air turbine water injection electromagnetic valve 52a (supply on-off valve 40a). Supply tube 20b through which water is fed to first air turbine handpiece 31 is provided with a first air turbine water injection switch electromagnetic valve 52b (supply tube on-off valve 40b).

One end (a first end) of emission tube 20c of main tube 21 of first air turbine handpiece 31 is connected to first branch drain tube 72 at first main tube attachment and removal portion 10a1 of treatment main unit 10. The other end (a second end) of emission tube 20c of main tube 21 of first air turbine handpiece 31 is connected to supply tube 20b of main tube 21 of first air turbine handpiece 31 before first air turbine water injection switch electromagnetic valve 52b (supply tube on-off valve 40b).

First air turbine water injection switch electromagnetic valve 52b is arranged between a portion of connection between the other end of emission tube 20c of main tube 21 of first air turbine handpiece 31 and supply tube 20b and first air turbine handpiece 31. First air turbine water injection switch electromagnetic valve 52b is arranged on the downstream side of the portion of connection between the other end of emission tube 20c of first air turbine main tube 21 and supply tube 20b. Specifically, first air turbine water injection switch electromagnetic valve 52b (supply tube on-off valve 40b) is desirably provided in a portion of connection of medium conduit 20 to treatment handpiece 31.

Second branch water feed tube 53 is connected to second air turbine handpiece 32 with a main tube 22 (medium conduit 20) of second air turbine handpiece 32 being interposed. One end (a first end) of supply tube 20b of main tube 22 of second air turbine handpiece 32 is connected to second branch water feed tube 53 at a second main tube attachment and removal portion 10a2 of treatment main unit 10. The other end (a second end) of supply tube 20b of main tube 22 of second air turbine handpiece 32 is connected to second air turbine handpiece 32. Second branch water feed tube 53 is provided with a second air turbine water injection electromagnetic valve 53a (supply on-off valve 40a). Supply tube 20b through which water is fed to second air turbine handpiece 32 is provided with a second air turbine water injection switch electromagnetic valve 53b (supply tube on-off valve 40b).

One end (a first end) of emission tube 20c of main tube 22 of second air turbine handpiece 32 is connected to second branch drain tube 73 at second main tube attachment and removal portion 10a2 of treatment main unit 10. The other end (a second end) of emission tube 20c of main tube 22 of second air turbine handpiece 32 is connected to supply tube 20b of main tube 22 of second air turbine handpiece 32 before second air turbine water injection switch electromagnetic valve 53b (supply tube on-off valve 40b).

Second air turbine water injection switch electromagnetic valve 53b is arranged between a portion of connection between the other end of emission tube 20c of main tube 22 of second air turbine handpiece 32 and supply tube 20b and second air turbine handpiece 32. Second air turbine water injection switch electromagnetic valve 53b is arranged on the downstream side of the portion of connection between the other end of emission tube 20c of main tube 22 of second air turbine handpiece 32 and supply tube 20b. Specifically, second air turbine water injection switch electromagnetic valve 53b (supply tube on-off valve 40b) is desirably provided in a portion of connection of medium conduit 20 to treatment handpiece 32.

Third branch water feed tube 54 is connected to micromotor handpiece 33 with a main tube 23 (medium conduit 20) of micromotor handpiece 33 being interposed. One end (a first end) of supply tube 20b of main tube 23 of micromotor handpiece 33 is connected to third branch water feed tube 54 at a third main tube attachment and removal portion 10a3 of treatment main unit 10. The other end (a second end) of supply tube 20b of main tube 23 of micromotor handpiece 33 is connected to micromotor handpiece 33. Third branch water feed tube 54 is provided with a micromotor water injection electromagnetic valve 54a (supply on-off valve 40a). Supply tube 20b through which water is fed to micromotor handpiece 33 is provided with a micromotor water injection switch electromagnetic valve 54b (supply tube on-off valve 40b).

One end (a first end) of emission tube 20c of main tube 23 of micromotor handpiece 33 is connected to third branch drain tube 74 at third main tube attachment and removal portion 10a3 of treatment main unit 10. The other end (a second end) of emission tube 20c of main tube 23 of micromotor handpiece 33 is connected to supply tube 20b of main tube 23 of micromotor handpiece 33 before micromotor water injection switch electromagnetic valve 54b (supply tube on-off valve 40b). Though emission tube 20c is generally not provided in micromotor handpiece 33, it is provided in the present invention for cleaning of a tube.

Micromotor water injection switch electromagnetic valve 54b is arranged between a portion of connection between the other end of emission tube 20c of main tube 23 of micromotor handpiece 33 and supply tube 20b and micromotor handpiece 33. Micromotor water injection switch electromagnetic valve 54b is arranged on the downstream side of the portion of connection between the other end of emission tube 20c of main tube 23 of micromotor handpiece 33 and supply tube 20b. Specifically, micromotor water injection switch electromagnetic valve 54b (supply tube on-off valve 40b) is desirably provided in a portion of connection of medium conduit 20 to treatment handpiece 33.

Fourth branch water feed tube 55 is connected to three-way syringe 34 with a main tube 24 (medium conduit 20) of three-way syringe 34 being interposed. One end (a first end) of supply tube 20b of main tube 24 of three-way syringe 34 is connected to fourth branch water feed tube 55 at a fourth main tube attachment and removal portion 10a4 of treatment main unit 10. The other end (a second end) of supply tube 20b of main tube 24 of three-way syringe 34 is connected to three-way syringe 34. Three-way syringe 34 includes a manual on-off valve (a control lever for water feed) 34b and is configured such that manual on-off valve (control lever for water feed) 34b is opened by an operation to press on-off valve (control lever for water feed) 34b and water is injected from a tip end portion 34c of three-way syringe 34.

One end (a first end) of emission tube 20c through which water in main tube 24 of three-way syringe 34 is emitted is connected to second main drain tube 75 at fourth main tube attachment and removal portion 10a4 of treatment main unit 10. The other end (a second end) of emission 20c through which water in main tube 24 of three-way syringe 34 is emitted is connected to supply tube 20b of main tube 24 of three-way syringe 34. In three-way syringe 34, a portion of branch of supply tube 20b and emission tube 20c is desirably disposed in the vicinity of a portion of connection of medium conduit 20 to three-way syringe 34.

First main drain tube 71 is provided with a main tube flushing electromagnetic valve (emission on-off valve) 40c. Main tube flushing electromagnetic valve (emission on-off valve) 40c is arranged between cleaning tank 130 and a merge portion where first branch drain tube 72, second branch drain tube 73, and third branch drain tube 74 merge with first main drain tube 71. Main tube flushing electromagnetic valve (emission on-off valve) 40c is arranged on the downstream side of the merge portion where first branch drain tube 72, second branch drain tube 73, and third branch drain tube 74 merge with first main drain tube 71.

Second main drain tube 75 is provided with a main conduit flushing electromagnetic valve 40d. Second main drain tube 75 is connected to cleaning tank (a drainage water container) 130. Cleaning tank 130 is configured to store water emitted from emission tube 20c through which water in first air turbine main tube 21, second air turbine main tube 22, micromotor main tube 23, and three-way syringe main tube 24 is emitted.

Cleaning tank 130 and drain portion 140 are connected to each other through a connection tube 141. Drain portion 140 is configured such that water is emitted from emission tube 20c of medium conduit 20. Cleaning tank 130 and vacuum tank 150 are connected to each other through a connection tube 151. Medical device 100 in the present embodiment is configured such that water stored in cleaning tank 130 flows to vacuum tank 150 connected to cleaning tank 130 to clean the vacuum tank.

While first air turbine handpiece 31, second air turbine handpiece 32, and micromotor handpiece 33 are normally used, a sensor (not shown) provided in a holder detects pick-up of at least any instrument from the holder in treatment main unit 10. When a foot switch (not shown) is operated, supply on-off valve 40a and supply tube on-off valve 40b corresponding to the removed instrument are opened and emission on-off valve 40c is closed, so that water is injected through an injection port of the instrument.

During flushing (cleaning) of each treatment handpiece 30, supply on-off valve 40a corresponding to at least any instrument is opened, supply tube on-off valve 40b is closed, and emission on-off valve 40c is opened so that water is emitted from supply tube 20b of medium conduit 20 via emission tube 20c and drainage water tube 70 to cleaning tank 130.

Fourth branch water feed tube 55 connected to three-way syringe 34 is not provided with supply on-off valve 40a. Supply tube 20b of main tube 24 of three-way syringe 34 is not provided with supply tube on-off valve 40b. Three-way syringe 34 includes control lever for water feed 34b in a grip portion 34a. As a doctor and an assistant perform an operation to press control lever 34b for water feed, water is injected from a tip end nozzle portion 34c of three-way syringe 34. Unless an operation to press control lever for water feed 34b is performed, water is not injected from three-way syringe 34. Therefore, though supply tube 20b of three-way syringe main tube 24 is not provided with supply tube on-off valve 40b, it is provided with emission tube 20c and drainage water tube (second main drain tube) 75. Therefore, water in the tube is emitted from supply tube 20b of medium conduit 20 via emission tube 20c and drainage water tube (second main drain tube) 75 to cleaning tank 130.

Fifth branch water feed tube 56 is connected to cup water faucet 2c. Fifth branch water feed tube 56 is provided with a cup water feed valve 56a. For example, an electromagnetic valve is employed for cup water feed valve 56a. When a sensor (not shown) provided in cup water faucet 2c detects presence of a cup (not shown) under cup water faucet 2c, cup water feed valve 56a is opened so that water is fed from cup water faucet 2c into the cup.

Air supply tube 60 includes a main air supply tube 61, a first branch air supply tube 62, a second branch air supply tube 63, a third branch air supply tube 64, and a fourth branch air supply tube 65. First branch air supply tube 62, second branch air supply tube 63, third branch air supply tube 64, and fourth branch air supply tube 65 are branched from main air supply tube 61.

Main air supply tube 61 is connected to an air source 60a serving as a compression air source by means of a compressor (not shown). Main air supply tube 61 is provided with a manual source valve 61a and a sterilization filter 61b sequentially from the upstream side.

First branch air supply tube 62 is connected to first air turbine handpiece 31 with first air turbine main tube (medium conduit) 21 being interposed. First branch air supply tube 62 is provided with a first air turbine driving air electromagnetic valve 62a. First air turbine main tube 21 has air feed tube 20d. One end (a first end) of air feed tube 20d of first air turbine main tube 21 is connected to first branch air supply tube 62 at first main tube attachment and removal portion 10a1 of treatment main unit 10. The other end (a second end) of air feed tube 20d of first air turbine main tube 21 is connected to first air turbine handpiece 31 so that a turbine (not shown) provided in an air turbine handpiece head (not shown) can be rotated or air can be injected through a not-shown separate injection port.

Second branch air supply tube 63 is connected to second air turbine handpiece 32 with second air turbine main tube (medium conduit) 22 being interposed. Second branch air supply tube 63 is provided with a second air turbine driving air electromagnetic valve 63a. Second air turbine main tube 22 has air feed tube 20d. One end (a first end) of air feed tube 20d of second air turbine main tube 22 is connected to second branch air supply tube 63 at second main tube attachment and removal portion 10a2 of treatment main unit 10. The other end (a second end) of air feed tube 20d of second air turbine main tube 22 is connected to second air turbine handpiece 32 so that a turbine (not shown) provided in an air turbine handpiece head (not shown) can be rotated or air can be injected through a not-shown separate injection port.

Third branch air supply tube 64 is connected to micromotor handpiece 33 with micromotor main tube (medium conduit) 23 being interposed. Third branch air supply tube 64 is provided with a micromotor cooling air electromagnetic valve 64a. Micromotor main tube 23 has air feed tube 20d. One end (a first end) of air feed tube 20d of micromotor main tube 23 is connected to third branch air supply tube 64 at third main tube attachment and removal portion 10a3 of treatment main unit 10. The other end (a second end) of air feed tube 20d of micromotor main tube 23 is connected to micromotor handpiece 33 so that air can be injected through a not-shown separate injection port.

Fourth branch air supply tube 65 is connected to three-way syringe 34 with three-way syringe main tube (medium conduit) 24 being interposed. Three-way syringe 34 has air feed tube 20d. One end (a first end) of air feed tube 20d of three-way syringe main tube 24 is connected to fourth branch air supply tube 65 at fourth main tube attachment and removal portion 10a4 of treatment main unit 10. The other end (a second end) of air feed tube 20d of three-way syringe main tube 24 is connected to three-way syringe 34.

A handpiece control block 10b includes first air turbine water injection electromagnetic valve 52a, second air turbine water injection electromagnetic valve 53a, micromotor water injection electromagnetic valve 54a, first air turbine driving air electromagnetic valve 62a, second air turbine driving air electromagnetic valve 63a, and micromotor cooling air electromagnetic valve 64a.

As a foot switch (not shown) is operated, first air turbine driving air electromagnetic valve 62a and second air turbine driving air electromagnetic valve 63a corresponding to at least any instrument of first air turbine handpiece 31 and second air turbine handpiece 32 are opened and closed, so that turbine-driving air is supplied to first air turbine handpiece 31 and second air turbine handpiece 32 to rotate turbines (not shown) contained in air turbine heads (not shown) or can be injected through not-shown separate injection ports.

As a foot switch (not shown) is operated, micromotor cooling air electromagnetic valve 64a is opened and closed so that cooling air is supplied to micromotor handpiece 33 and injected through a not-shown separate injection port of micromotor handpiece 33.

Fourth branch air supply tube 65 is not provided with an electromagnetic valve as above. Three-way syringe 34 includes a control lever for air (not shown) in grip portion 34a. As a doctor and an assistant perform an operation to press the control lever for air, air is injected from tip end nozzle portion 34c. The control lever for air is juxtaposed with control lever for water feed 34b. A doctor and an assistant selectively or simultaneously perform an operation to press the control lever for air and control lever for water feed 34b.

Cleaning device 110 is connected to main water feed tube 51. When a medicinal solution cleaning device is employed as cleaning device 110, the medicinal solution cleaning device includes a medicinal solution bottle 110a and a medicinal solution pump 110b. Medicinal solution bottle 110a is filled, for example, with a hydrogen peroxide solution or a medicinal solution of sodium hypochlorite or the like. Medicinal solution pump 110b is configured to dispense the medicinal solution in medicinal solution bottle 110a to main water feed tube 51. A medicinal solution amount detection portion 111 is attached to medicinal solution bottle 110a. A sensor which can detect an amount of medicinal solution in medicinal solution bottle 110a is employed for medicinal solution amount detection portion 111.

Drainage water from cleaning tank 130 is drained through connection tube 141 to drain portion 140. Drainage water from cleaning tank 130 is sent to vacuum tank 150 via connection tube 151. Vacuum tank 150 can be cleaned with this drainage water.

Control unit 160 is configured to be responsible for overall control of medical device 100. For example, a central processing unit (CPU) is employed for control unit 160. Cleaning device 110, medicinal solution amount detection portion 111, flowmeter 120, a drive portion 161, a display portion 162, a notification portion 163, a tube cleaning switch 164, a flushing switch 165, a main switch 166, and water quality monitoring detector 170 are connected to control unit 160.

Drive portion 161 serves to drive an electromagnetic valve or the like of each instrument or the like. Display portion 162 is set on tray table 5 or the like and shows various types of information on treatment or the like. Notification portion 163 functions to give a notification when decrease in amount of a medicinal solution in the medicinal solution bottle of cleaning device 110 is detected by medicinal solution amount detection portion 111 and an abnormal condition of another device is sensed.

In another example, treatment main unit 10 may include a microbubble generator as cleaning device 110 or may sterilize a tube with an ultraviolet ray generator. In the example above, drainage water is stored in cleaning tank 130 and then drained. Water in the tube, however, can also be circulated by connecting drain tube 71 and 75 and supply tube 20b to one another before cleaning tank 130 with a pump and a three-way electromagnetic valve which are not shown being interposed and driving this pump. When water in a tube is circulated, a medicinal solution cleaning device, an ultraviolet ray generator, or a microbubble generator is used to circulate water for a prescribed period of time approximately from 1 to 5 minutes and thereafter water in the tube is drained to cleaning tank 130 through the three-way valve, so that water in the tube can efficiently be cleaned with a small amount of water. An electromagnetic valve may be employed for supply tube on-off valve 40b shown in Fig. 4, and electric power is supplied by providing a not-shown wire as being connectable from treatment main unit 10 with the use of a pin or the like in order to open and close the electromagnetic valve.

Tube cleaning switch 164, flushing switch 165, and main switch 166 shown in Fig. 5 are provided in tray table 5 (see Fig. 1) or the like of treatment main unit 10. Tube cleaning switch 164 is operated by a doctor, an assistant, and the like in cleaning of a water feed tube. Flushing switch 165 is operated by a doctor, an assistant, and the like in cleaning of the water feed tube prior to treatment with medical device 100. Main switch 166 is operated by a doctor, an assistant, and the like when medical device 100 is started up. Supply on-off valve 40a, supply tube on-off valve 40b, emission on-off valve 40c, and main conduit flushing electromagnetic valve 40d may be configured to be activated in coordination with activation of main switch 166 of treatment main unit 10. Control unit 160 may control drive portion 161 so as to automatically activate supply on-off valve 40a, supply tube on-off valve 40b, emission on-off valve 40c, and main conduit flushing electromagnetic valve 40d in response to an output from water quality monitoring detector 170. In response to an output from water quality monitoring detector 170, flushing may automatically be driven and controlled by means of a timer so as to be completed after a prescribed period of time approximately from 1 to 5 minutes before actual treatment time.

A method of cleaning medical device 100 according to the present embodiment will now be described with reference to Figs. 4 to 7.

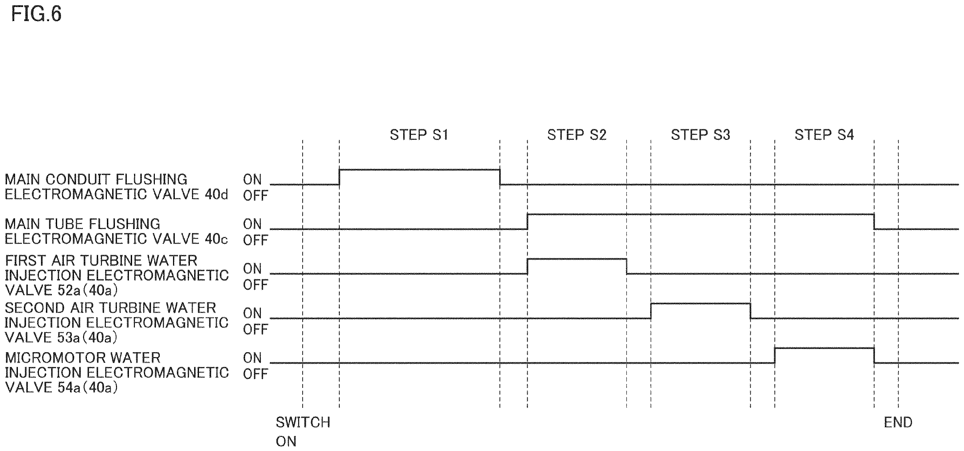

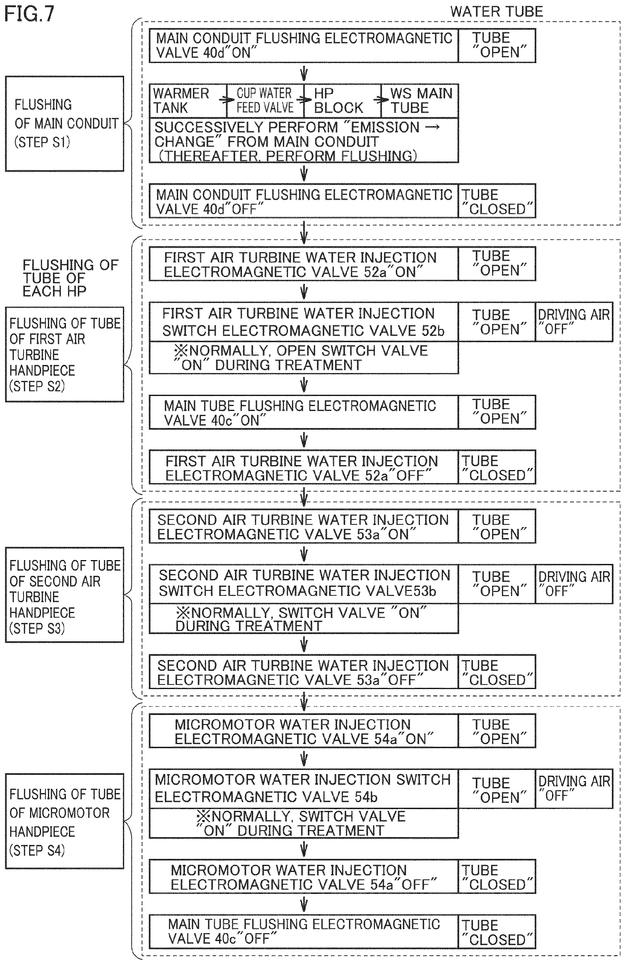

Referring mainly to Figs. 6 and 7, when main switch 166 or flushing switch 165 is turned on (switch on), flushing of the main conduit for cleaning the three-way syringe is performed. Initially, main conduit flushing electromagnetic valve 40d for cleaning the three-way syringe is turned on (open) (step S1). Thus, the main conduit (drain tube) for cleaning the three-way syringe is opened (open), and supply tube on-off valves 40b in the supply tubes (water feed tubes) to the turbines and the micromotor remain closed. In succession, when water feed electromagnetic valve 51b is turned on to open, water is fed through the supply tube in the order of warmer tank 51d, handpiece control block (HP block) 10b, and three-way syringe main tube (WS main tube) 24. Thus, remaining water is emitted sequentially from the main conduit for cleaning the three-way syringe and change with fresh water is performed. Subsequently, flushing of the tube of each treatment handpiece is performed. In succession, main conduit flushing electromagnetic valve 40d is turned off (closed). The tube is thus closed (closed).

Then, flushing of the tube of each treatment handpiece (HP) other than the three-way syringe is performed.

Initially, flushing of the tube of the first air turbine handpiece is performed (step S2). First air turbine water injection electromagnetic valve 52a (40a) is turned on (open). The tube is thus opened. In succession, first air turbine water injection switch electromagnetic valve 52b (40b) is normally off (closed) except for during treatment. In this state, a water injection supply tube to a handpiece tip end portion is closed. Normally, during treatment, first air turbine water injection switch electromagnetic valve 52b is on (open). When a signal indicating pick-up from a handpiece holder and an operation signal from a foot controller are received, first air turbine handpiece 31 is driven and water is injected into the handpiece tip end portion. During flushing, however, first air turbine water injection switch electromagnetic valve 52b (40b) is turned off (closed). In succession, main tube flushing electromagnetic valve 40c is turned on. In this state, the tube is open. Therefore, water flows through the tube in the order of main water feed tube 51, first branch water feed tube 52, supply tube 20b of first air turbine handpiece 31, emission tube 20c, first branch drain tube 72, and first main drain tube 71, and drained to cleaning tank 130. The tube is thus cleaned with tap water. Therefore, supply tube 20b of first air turbine handpiece 31 is cleaned. In succession, after lapse of a prescribed period of time necessary for cleaning of the tube, first air turbine water injection electromagnetic valve 52a (40a) is turned off. The tube is thus closed.

Then, flushing of the tube of the second air turbine handpiece is performed (step S3). Second air turbine water injection electromagnetic valve 53a (40a) is turned on. The tube is thus opened. In succession, second air turbine water injection switch electromagnetic valve 53b (40b) is normally off except for during treatment. In this state, a supply tube to the handpiece is closed. Normally, during treatment, second air turbine water injection switch electromagnetic valve 53b is on (open). Main tube flushing electromagnetic valve 40c is on (open). Therefore, water flows through the tube in the order of main water feed tube 51, second branch water feed tube 53, supply tube 20b of second air turbine handpiece 32, emission tube 20c, second branch drain tube 73, and first main drain tube 71 and is drained to cleaning tank 130. The tube is thus cleaned. Therefore, supply tube 20b of second air turbine handpiece 32 is cleaned. In succession, after lapse of a prescribed period of time necessary for cleaning of the tube, second air turbine water injection electromagnetic valve 53a is turned off. The tube is thus closed.

In succession, flushing of the tube of the micromotor handpiece is performed (step S4).

Micromotor water injection electromagnetic valve 54a (40a) is turned on. The tube is thus open. In succession, micromotor water injection switch electromagnetic valve 54b (40b) is normally off except for during treatment. In this state, the supply tube to the handpiece is closed. Normally, during treatment, micromotor water injection switch electromagnetic valve 54b (40b) is turned on and the tube is open. Main tube flushing electromagnetic valve 40c is on (open). Therefore, water flows through the tube in the order of main water feed tube 51, third branch water feed tube 54, supply tube 20b of micromotor handpiece 33, emission tube 20c, third branch drain tube 74, and first main drain tube 71 and is drained into cleaning tank 130. The tube is thus cleaned. Therefore, supply tube 20b of micromotor handpiece 33 is cleaned. In succession, micromotor water injection electromagnetic valve 54a (40a) is turned off. The tube is thus closed. In succession, main tube flushing electromagnetic valve 40c is turned off. The tube is thus closed. Flushing of the tube of each treatment handpiece thus ends. Treatment handpieces are successively cleaned from a tube higher in flow rate. Thus, water can quickly be fed to tubes in the supply tube limited in volume. Therefore, the inside of the medium conduit can efficiently be cleaned with a small amount of water. By performing this flushing operation for a prescribed period of time approximately from 1 to 5 minutes, the tubes are disinfected and cleaned with chlorine in tap water and hence water is safe to drink. Though main tube flushing electromagnetic valve 40c is turned on in step S2 and turned off in step S4 in the above example, it may be turned on and off for each of step S2 to step S4.

This operation may be started automatically a prescribed time before the doctor's hours every morning after main switch 166 is turned on. Alternatively, this operation may automatically be started when a concentration of chlorine is lowered or the like. Alternatively, this operation may automatically be started by means of a timer at the set time before treatment.

Supply on-off valve 40a and supply tube on-off valve 40b may be activated automatically in response to an output from water quality monitoring detector 170. Thus, automatic cleaning can be performed when water is deteriorated.

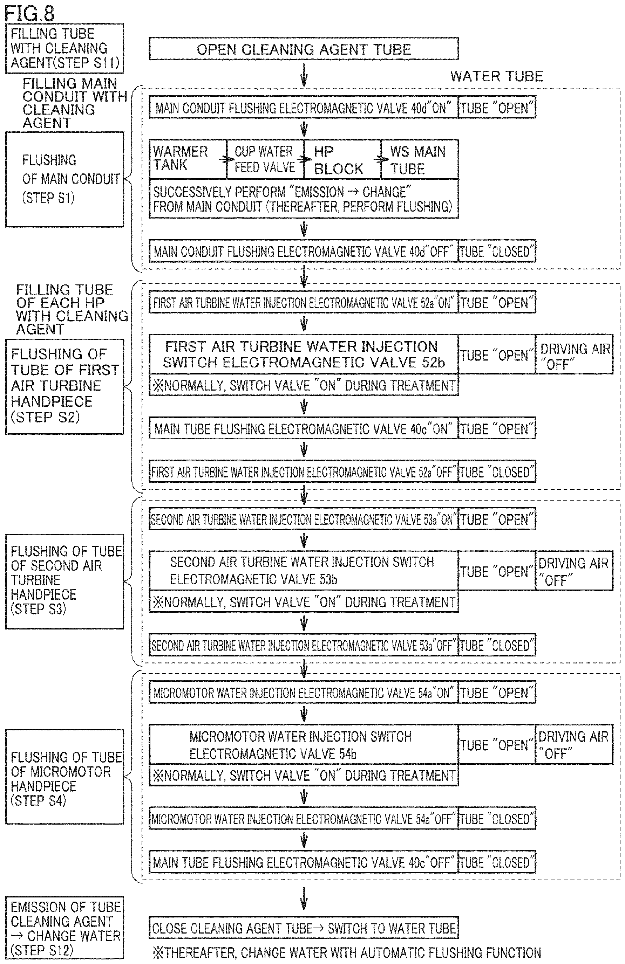

Referring to Fig. 8, main water feed tube 51 may be filled with a tube cleaning agent (a medicinal solution) via a cleaning agent tube from cleaning device 110. When the cleaning agent tube is opened, the main conduit is filled with the cleaning agent (step S11). Flushing of the main conduit (step S1), flushing of the tube of the first air turbine handpiece (step S2), flushing of the tube of the second air turbine handpiece (step S3), and flushing of the tube of the micromotor handpiece (step S4) are successively performed with water filled with the cleaning agent. When flushing of the tube of each treatment handpiece ends, in succession, the cleaning agent tube is closed and switching to a water tube is made (step S12). Water is changed thereafter with an automatic flushing function.

This invention is fundamentally different from the prior art in Japanese Patent No. 4348075 in that after a tube is cleaned with a medicinal solution, the tube is again cleaned with tap water. By replacing a medicinal solution with tap water, influence by the medicinal solution which remains in the tube can also be eliminated.

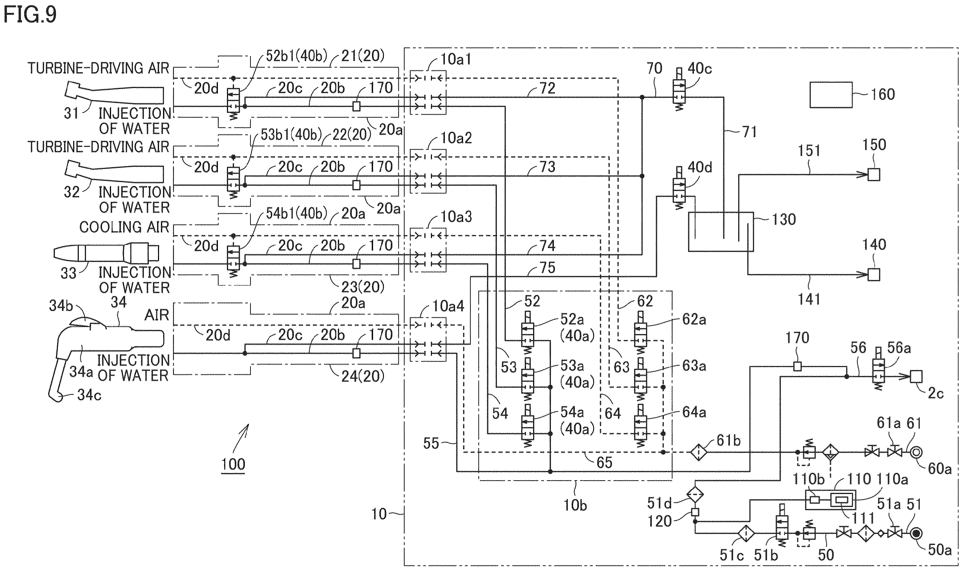

In succession, a first modification of the present embodiment will be described with reference to Fig. 9. The first modification of the present embodiment is the same in configuration as the present embodiment unless otherwise mentioned, and the same feature has the same reference allotted and description thereof will not be repeated.

In the first modification of the present embodiment, unlike the electromagnetic valve above, supply tube on-off valve 40b is configured to be activated by air.

Supply tube 20b of first air turbine handpiece 31 is provided with a first air turbine water injection switch valve 52b1 (supply tube on-off valve 40b). First air turbine water injection switch valve 52b1 is not an electromagnetic valve but is configured to be operated by turbine-driving air. Supply tube 20b of second air turbine handpiece 32 is provided with a second air turbine water injection switch valve 53b1 (supply tube on-off valve 40b). Second air turbine water injection switch valve 53b1 is not an electromagnetic valve but is configured to be operated by turbine-driving air. Supply tube 20b of micromotor handpiece 33 is provided with a micromotor water injection switch valve 54b1 (supply tube on-off valve 40b). Micromotor water injection switch valve 54b1 is not an electromagnetic valve but is configured to be operated by cooling air.

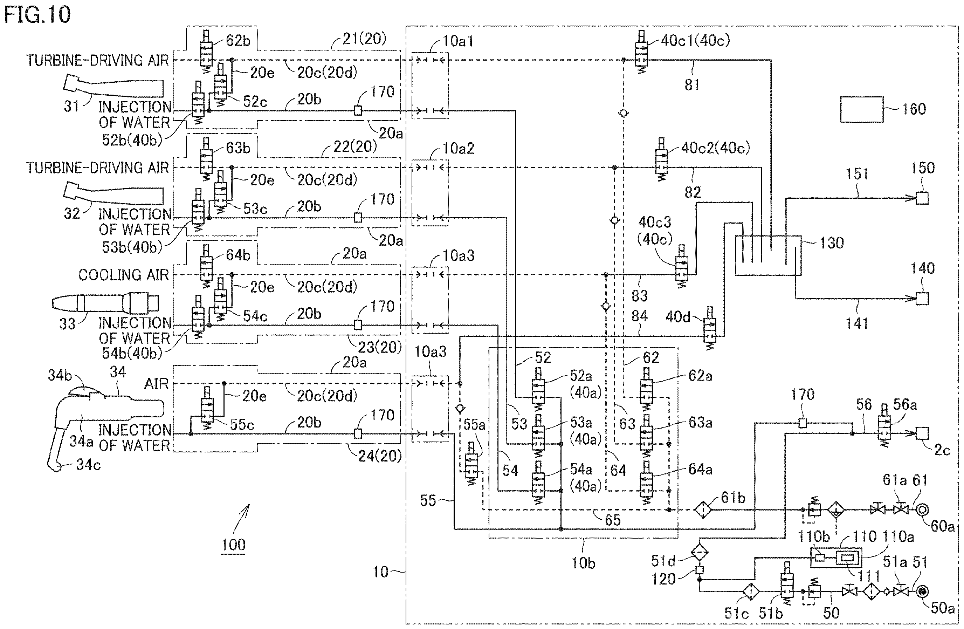

In succession, a second modification of the present embodiment will be described with reference to Fig. 10. The second modification of the present embodiment is the same in configuration as the present embodiment unless otherwise mentioned, and the same feature has the same reference allotted and description thereof will not be repeated. In the second modification of the present embodiment, water is drained through supply tube 20b via air feed tube 20d. In the second modification of the present embodiment, air feed tube 20d through which air is fed from treatment main unit 10 to treatment handpiece 30 serves as emission tube 20c.

Air feed tube 20d of first air turbine handpiece 31 is provided with an air feed tube electromagnetic valve 62b. Supply tube 20b and air feed tube 20d of first air turbine handpiece 31 are connected to each other through an air feed bypass tube 20e. Air feed bypass tube 20e is provided with an air feed bypass tube electromagnetic valve 52c. During cleaning, water can be fed from supply tube 20b of first air turbine handpiece 31 through air feed bypass tube 20e to air feed tube 20d by closing air feed tube electromagnetic valve 62b and opening air feed bypass tube electromagnetic valve 52c. Air feed p tube 20d of first air turbine handpiece 31 is connected to a first emission tube 81. First emission tube 81 is provided with a first emission tube electromagnetic valve 40c1 (emission on-off valve 40c). First emission tube 81 is connected to cleaning tank 130.

Air feed tube 20d of second air turbine handpiece 32 is provided with an air feed tube electromagnetic valve 63b. Supply tube 20b and air feed tube 20d of second air turbine handpiece 32 are connected to each other through air feed bypass tube 20e. Air feed bypass tube 20e is provided with an air feed bypass tube electromagnetic valve 53c. During cleaning, water can be fed from supply tube 20b of second air turbine handpiece 32 through air feed bypass tube 20e to air feed tube 20d by closing air feed tube electromagnetic valve 63b and opening air feed bypass tube electromagnetic valve 53c. Air feed tube 20d of second air turbine handpiece 32 is connected to a second emission tube 82. Second emission tube 82 is provided with a second emission tube electromagnetic valve 40c2 (emission on-off valve 40c). Second emission tube 82 is connected to cleaning tank 130.

Air feed tube 20d of micromotor handpiece 33 is provided with an air feed tube electromagnetic valve 64b. Supply tube 20b and air feed tube 20d of micromotor handpiece 33 are connected to each other through air feed bypass tube 20e. Air feed bypass tube 20e is provided with an air feed bypass tube electromagnetic valve 54c. During cleaning, water can be fed from supply tube 20b of micromotor handpiece 33 through air feed bypass tube 20e to air feed tube 20d by closing air feed tube electromagnetic valve 64b and opening air feed bypass tube electromagnetic valve 54c. Air feed tube 20d of micromotor handpiece 33 is connected to a third emission tube 83. Third emission tube 83 is provided with a third emission tube electromagnetic valve 40c3 (emission on-off valve 40c). Third emission tube 83 is connected to cleaning tank 130.

Supply tube 20b and air feed tube 20d of three-way syringe 34 are connected to each other through air feed bypass tube 20e. Air feed bypass tube 20e is provided with an air feed bypass tube electromagnetic valve 55c. During cleaning, water can be fed from supply tube 20b of three-way syringe 34 through air feed bypass tube 20e to air feed tube 20d by opening air feed bypass tube electromagnetic valve 55c. Air feed tube 20d of three-way syringe 34 is connected to a fourth emission tube 84. Fourth emission tube 84 is provided with main conduit flushing electromagnetic valve 40d. Fourth emission tube 84 is connected to cleaning tank 130. Fourth branch air supply tube 65 is provided with an electromagnetic valve 55a for stopping air feed during cleaning of the tube when water is drained through air feed tube 20d of three-way syringe 34.

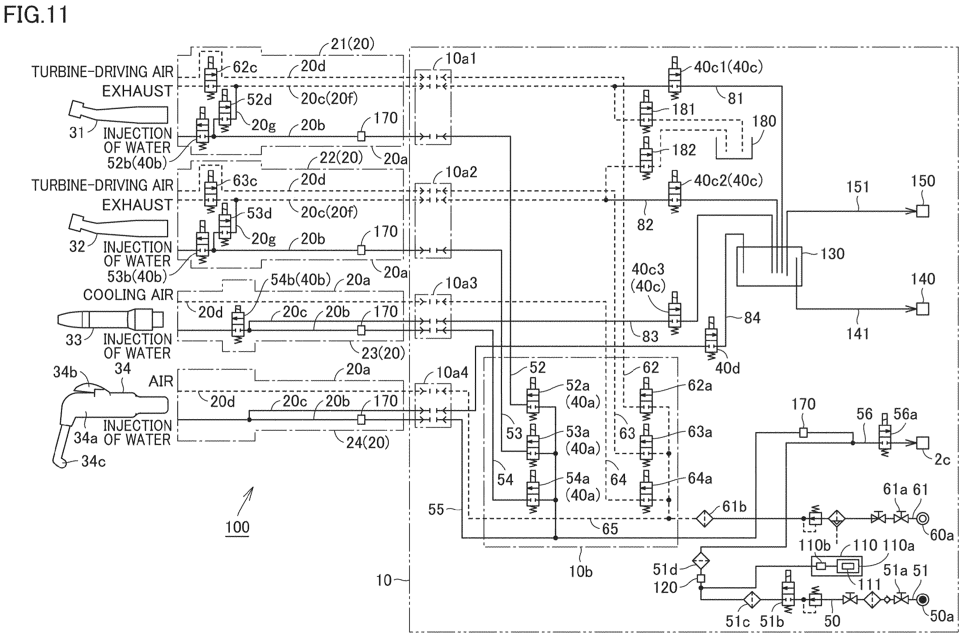

In succession, a third modification of the present embodiment will be described with reference to Fig. 11. The third modification of the present embodiment is the same in configuration as the present embodiment and the second modification unless otherwise mentioned, and the same feature has the same reference allotted and description thereof will not be repeated.

In the third modification of the present embodiment, water is drained through supply tube 20b via exhaust tube 20f. In the third modification of the present embodiment, exhaust tube 20f through which air is exhausted from treatment handpiece 30 to treatment main unit 10 serves as emission tube 20c. A drain construction in which exhaust tube 20f is used can be adopted only for an air turbine handpiece which originally includes an exhaust tube, and water is drained through emission tube 20c in the micromotor handpiece or the three-way syringe.

First air turbine main tube 21 includes exhaust tube 20f. In the air turbine handpiece, air supplied through the air feed tube is supplied to a handpiece head (not shown) to drive the turbine, and thereafter released in the treatment main unit via exhaust tube 20f.

One end (a first end) of exhaust tube 20f of first air turbine main tube 21 is connected to first emission tube 81 at first main tube attachment and removal portion 10a1. The other end (a second end) of exhaust tube 20f of first air turbine main tube 21 is connected to first air turbine handpiece 31.

An exhaust tube electromagnetic valve 62c is provided in exhaust tube 20f of first air turbine main tube 21. Supply tube 20b and exhaust tube 20f of first air turbine handpiece 31 are connected to each other through an exhaust bypass tube 20g. Exhaust bypass tube 20g is provided with an exhaust bypass tube electromagnetic valve 52d. During cleaning, water can be fed from supply tube 20b of first air turbine handpiece 31 through exhaust bypass tube 20g to exhaust tube 20f by closing exhaust tube electromagnetic valve 62c provided in handpiece 31, opening exhaust bypass tube electromagnetic valve 52d provided in handpiece 31, opening on-off valve 52a provided in handpiece control block 10b, closing on-off valve 52b provided in handpiece 31, closing an on-off valve 181 provided in treatment main unit 10, and opening on-off valve 40c1 provided in treatment main unit 10.

Second air turbine main tube 22 includes exhaust tube 20f. One end (a first end) of exhaust tube 20f of second air turbine main tube 22 is connected to second emission tube 82 at second main tube attachment and removal portion 10a2. The other end (a second end) of exhaust tube 20f of second air turbine main tube 22 is connected to second air turbine handpiece 32.

An exhaust tube electromagnetic valve 63c is provided in exhaust tube 20f of second air turbine main tube 22. Supply tube 20b and exhaust tube 20f of second air turbine handpiece 32 are connected to each other through exhaust bypass tube 20g. Exhaust bypass tube 20g is provided with an exhaust bypass tube electromagnetic valve 53d. During cleaning, water can be fed from supply tube 20b of second air turbine handpiece 32 through exhaust bypass tube 20g to exhaust tube 20f by closing exhaust tube electromagnetic valve 63c provided in handpiece 32, opening exhaust bypass tube electromagnetic valve 53d provided in handpiece 32, opening on-off valve 53a provided in handpiece control block 10b, closing on-off valve 53b provided in handpiece 32, closing an on-off valve 182 provided in treatment main unit 10, and opening on-off valve 40c2 provided in treatment main unit 10.

Emission tube 20c of first air turbine handpiece 31 and emission tube 20c of second air turbine handpiece 32 are branched before on-off valve 40c1 and on-off valve 40c2, respectively, drainage water is emitted to the cleaning tank via tubes 81 and 82, and an exhaust is emitted to an oil collection case 180 through on-off valves 181 and 182 so that a lubricant is recovered therein.

A tube is cleaned by feeding the tube with tap water with a time period for flushing with tap water being normally set to a time period approximately from 1 to 5 minutes. A clock counting a time period during which a medical device is not used may be provided in the medical device, and when the time period counted by this clock during which the medical device has not been used is long, cleaning may be performed with a time period for flushing with tap water being set to be longer than 5 minutes so that germs in the tube are reliably killed. Thus, a length of the time period for flushing may be determined in accordance with a length of a time period during which the medical device has not been used. The clock may count time in coordination with on/off of a main switch or in coordination with a signal indicating pick-up of a treatment handpiece from a handpiece holder or an operation of a foot controller, and separately provided storage means may store a time period during which the medical device has been used and has not been used.

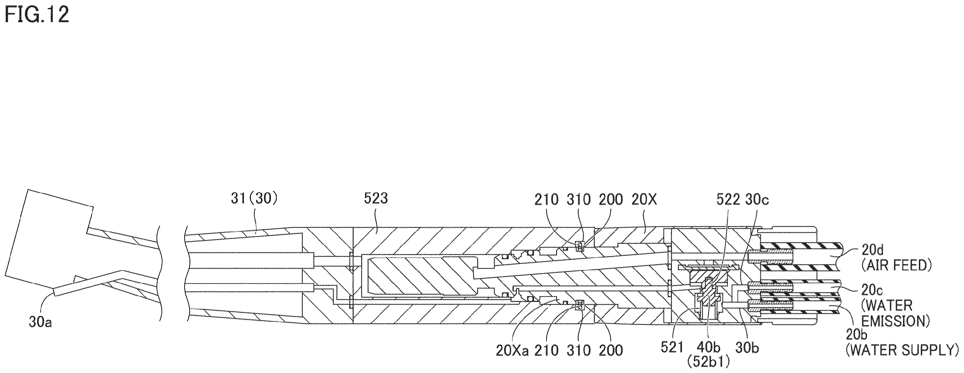

Fig. 12 is an assembly cross-sectional view of an engagement portion 20X provided at a tip end portion of medium conduit 20 for attachment to, removal from, and engagement with treatment handpiece 30 shown in Fig. 2. In engagement portion 20X, first air turbine water injection switch valve 52b1 (supply tube on-off valve 40b) described with reference to Fig. 9 is provided. When turbine-driving air is supplied to first air turbine water injection switch valve 52b1 (supply tube on-off valve 40b), a valve element 522 of supply tube on-off valve 40b is pressed downward against a spring 521 by an air feed pressure in air feed tube 20d, supply tube 20b is opened, air is fed to the handpiece, the handpiece is driven, and water is injected through an injection port provided in a handpiece head. While air is not supplied, valve element 522 is normally closed by force from spring 521. Therefore, when supply on-off valve 40a is opened, water from supply tube 20b is emitted toward emission tube 20c.

Air turbine handpiece 31 shown in Fig. 12 has an injection port 30a through which water is injected to the outside. Engagement portion 20X contains supply tube on-off valve 40b, a supply path 30b, and an emission path 30c, and this is also the case with the micromotor handpiece. The three-way syringe is not provided with supply tube on-off valve 40b but with a manual on-off valve, supply path 30b, and emission path 30c. When air feed tube 20d shown in the example in Fig. 10 is employed as emission tube 20c, three-way syringe 34 contains supply tube on-off valve 40b, air feed bypass electromagnetic valve 55c, supply tube 20b, and emission tube 20c. Examples of a type of the handpiece may include an ultrasonic scaler and an air scaler in addition thereto. In order to prevent handpiece 31 from inadvertently falling from engagement portion 20X of medium conduit 20, a groove 200 is provided around an outer circumference of a protrusion 20Xa of engagement portion 20X, a C ring 210 formed from an elastic body fitted into groove 200 is attached, and a circumferential groove 310 in which C ring 210 is fitted is provided around an inner circumference of a coupling portion 523 of corresponding handpiece 31. C ring 210 is slightly greater in outer diameter than protrusion 20Xa while C ring 210 is attached to groove 200 provided around the outer circumference of protrusion 20Xa. With elasticity of C ring 210, the diameter of C ring 210 is increased at the time of coupling so that engagement portion 20X and handpiece 31 are coupled to each other. While coupling portion 523 is coupled to protrusion 20Xa, handpiece 31 is held as not falling from engagement portion 20X by fitting into groove 310 having an outer diameter slightly greater than an inner diameter of an inner circumferential portion of coupling portion 523 and provided in the inner circumferential portion of coupling portion 523. Since the C ring has a cross-section in a form of an inclined peak, the outer diameter of the C ring is made smaller to a diameter equal to or smaller than an inner diameter of coupling portion 523 of handpiece 31 in groove 200 by axially pulling handpiece 31 with respect to engagement portion 20X and hence handpiece 31 and engagement portion 20X are decoupled from each other. Attachment and removal is thus easy. The C ring may be attached to groove 310 provided around the inner circumference of coupling portion 523.

A function and effect of the present embodiment will now be described.

According to medical device 100 in the present embodiment, valve 40 is configured to allow water to flow either to treatment handpiece 30 from supply tube 20b or to treatment main unit 10 via emission tube 20c so that water can be fed from supply tube 20b to emission tube 20c. Therefore, since supply tube 20b can be cleaned with water, cleaning of supply tube 20b can be simplified. Since the tube is sterilized by chlorine contained in tap water simply by feeding the tube with tap water without using a degerminating agent or without essentially requiring a degerminating device, growth of germs in the medium conduit can be suppressed. Therefore, "water" herein refers to tap water.

Medical device 100 in the present embodiment is preferably configured to allow water to flow from supply tube 20b via emission tube 20c to treatment main unit 10 by opening supply on-off valve 40a, closing supply tube on-off valve 40b, and opening main tube flushing electromagnetic valve 40c. Therefore, supply tube 20b can be cleaned by feeding water through supply tube 20b to emission tube 20c by opening supply on-off valve 40a and closing supply tube on-off valve 40b.

Medical device 100 in the present embodiment is preferably configured to allow water to flow from supply tube 20b of at least any of handpieces via emission tube 20c to treatment main unit 10 by controlling opening and closing of supply on-off valve 40a and supply tube on-off valve 40b provided for each handpiece and opening and closing main tube flushing electromagnetic valve 40c. Therefore, water can be fed from supply tube 20b to emission tube 20c for each handpiece and supply tube 20b can be cleaned by controlling opening and closing of supply on-off valve 40a and supply tube on-off valve 40b provided for each handpiece and main tube flushing electromagnetic valve 40c.

According to the first modification of medical device 100 in the present embodiment, supply tube on-off valve 40b is configured to be activated by air. Therefore, supply tube on-off valve 40b can be controlled with air. Therefore, supply tube on-off valve 40b can be controlled by using such air as turbine-driving air or cooling air. Specifically, an activation pressure of driving air in air feed tube 20d can be used as a signal pressure of a valve.

According to medical device 100 in the present embodiment, preferably, supply on-off valve 40a and supply tube on-off valve 40b are configured to be activated in coordination with activation of main switch 166 of treatment main unit 10. Therefore, water can be fed from supply tube 20b to emission tube 20c and supply tube 20b can be cleaned in coordination with activation of main switch 166. Therefore, a switch for cleaning does not have to particularly be operated. A switch for cleaning does not have to be provided.

According to medical device 100 in the present embodiment, preferably, supply on-off valve 40a and supply tube on-off valve 40b are configured to automatically be activated in response to an output from water quality monitoring detector 170. Therefore, when quality of water is equal to or lower than prescribed criteria, water quality monitoring detector 170 detects the quality so that water can automatically be fed from supply tube 20b to emission tube 20c and supply tube 20b can be cleaned. Therefore, quality of water in the tube being properly kept can be known.

According to medical device 100 in the present embodiment, preferably, a water feed tube through which water is supplied from treatment main unit 10 to treatment handpiece 30 serves as supply tube 20b.

According to medical device 100 in the present embodiment, preferably, a drain tube through which water is emitted from medium conduit 20 to treatment main unit 10 serves as emission tube 20c. Therefore, water can be drained through the drain tube.

Medical device 100 in the present embodiment is preferably configured to allow water stored in cleaning tank 130 to flow to vacuum tank 150. Therefore, vacuum tank 150 can be cleaned with water stored in cleaning tank 130. Therefore, water can effectively be used.

According to the second modification of medical device 100 in the present embodiment, valve 40 is configured to allow water to flow either to treatment handpiece 30 from supply tube 20b or to treatment main unit 10 to via air feed tube 20d. Therefore, air feed tube 20d can serve as emission tube 20c. Therefore, water can be fed from supply tube 20b to air feed tube 20d and supply tube 20b can be cleaned. Therefore, air feed tube 20d which has already been provided in treatment handpiece 30 can also serve as emission tube 20c. Namely, a special return tube does not have to be provided. Therefore, since the number of tubes does not increase, flexibility of medium conduit 20 can be prevented from being impaired. Furthermore, increase in cost can also be suppressed. Air feed tube 20d can be cleaned by feeding water.

According to the third modification of medical device 100 in the present embodiment, valve 40 is configured to allow water to flow either to treatment handpiece 30 from supply tube 20b or to treatment main unit 10 via exhaust tube 20f. Therefore, exhaust tube 20f can serve as emission tube 20c. Therefore, water can be fed from supply tube 20b to exhaust tube 20f and supply tube 20b can be cleaned. Therefore, exhaust tube 20f which has already been provided in treatment handpiece 30 can also serve as emission tube 20c. Namely, a special return tube does not have to be provided. Therefore, since the number of tubes does not increase, flexibility of medium conduit 20 can be prevented from being impaired. Furthermore, increase in cost can also be suppressed. Exhaust tube 20f can be cleaned by feeding water.

According to the medium conduit set in the present embodiment, valve 40 is configured to allow water to flow either to treatment handpiece 30 from supply tube 20b or to emission tube 20c. Therefore, water can be fed from supply tube 20b to emission tube 20c. Since supply tube 20b can be cleaned with water, cleaning of supply tube 20b can be simplified.

Though an embodiment of the present invention has been described, it should be understood that the embodiment disclosed herein is illustrative and non-restrictive in every respect. The scope of the present invention is defined by the terms of the claims and is intended to include any modifications within the scope of the claims.

## Claims

1. A medical device (100) comprising:
a treatment main unit (10);
a flexible medium conduit (20) connected to the treatment main unit (10);
a treatment handpiece (30) removably connected to the medium conduit (20) and removably connected to the treatment main unit (10) with the medium conduit (20) being interposed,
the medium conduit (20) including
an outer tube (20a),
a supply tube (20b) arranged in the outer tube (20a), the supply tube (20b) being configured to supply water from the treatment main unit (10) to the treatment handpiece (30), and
an emission tube (20c) arranged in the outer tube (20a) and connected to the supply tube (20b), the emission tube (20c) being configured to emit water from the medium conduit (20) to the treatment main unit (10); and
a valve arrangement configured to allow the water to flow from the treatment main unit (10) either to the supply tube (20b) and the treatment handpiece (30) or to the supply tube (20b) and back to the treatment main unit (10) via the emission tube (20c), wherein
the treatment main unit (10) includes a medium supply portion (51) configured to supply the water to the supply tube (20b),
the valve arrangement includes
a supply on-off valve (40a) arranged in the treatment main unit (10) between the medium supply portion (51) and the supply tube (20b), and
a supply tube on-off valve (40b) arranged in a portion of connection of the medium conduit (20) to the treatment handpiece (30) and between the treatment handpiece (30) and a connection portion of the supply tube (20b) to which the emission tube (20c) is connected,
the medical device (100) is configured to allow the water to flow from the treatment main unit (10) to the supply tube (20b) and via the emission tube (20c) back to the treatment main unit (10) by opening the supply on-off valve (40a) in the treatment main unit (10) and closing the supply tube on-off valve (40b),
the medium supply portion (51) is connected to a source of tap water (50a) and the supply tube (20b) is configured to supply tap water from the source of tap water (50a).

2. The medical device (100) according to claim 1, wherein
the treatment handpiece (30) includes a plurality of handpieces, and
the medical device (100) is configured to allow the water to flow from the supply tube (20b) of at least any of the handpieces via the emission tube (20c) to the treatment main unit (10) by controlling opening and closing of the supply on-off valve (40a) and the supply tube on-off valve (40b) provided for each handpiece.

3. The medical device (100) according to claim 1 or 2, wherein
the supply tube on-off valve (40b) is configured to be activated by air.

4. The medical device (100) according to any one of claims 1 to 3, wherein
the supply on-off valve (40a) and the supply tube on-off valve (40b) are configured to be activated in coordination with activation of a main switch (166) of the treatment main unit (10).

5. The medical device (100) according to any one of claims 1 to 4, the medical device (100) further comprising a detector (170) for monitoring quality of the water which remains in at least any of the treatment main unit (10) and the supply tube (20b), wherein
the supply on-off valve (40a) and the supply tube on-off valve (40b) are configured to automatically be activated in response to an output from the detector (170).

6. The medical device (100) according to any one of claims 1 to 5, wherein
the supply tube (20b) is a water feed tube through which the water is supplied from the treatment main unit (10) to the treatment handpiece (30).

7. The medical device (100) according to any one of claims 1 to 6, wherein
the emission tube (20c) is a drain tube through which the water is emitted from the medium conduit (20) to the treatment main unit (10).

8. The medical device (100) according to any one of claims 1 to 7, wherein
the treatment main unit (10) includes
a cleaning tank (130) which stores the water emitted from the emission tube (20c), and
a vacuum tank (150) connected to the cleaning tank (130),
the medical device (100) is configured to allow the water stored in the cleaning tank (130) to flow to the vacuum tank (150).

## Patentansprüche

1. Medizinische Vorrichtung (100), aufweisend:
eine Behandlungshaupteinheit (10);
eine flexible Medienleitung (20), die mit der Behandlungshaupteinheit (10) verbunden ist;
ein Behandlungshandstück (30), das mit der Medienleitung (20) abnehmbar verbunden ist und mit der Behandlungshaupteinheit (10) abnehmbar verbunden ist, wobei die Medienleitung (20) dazwischengeschaltet ist,
wobei die Medienleitung (20) umfasst
einen Außenschlauch (20a),
einen Zufuhrschlauch (20b), der im Außenschlauch (20a) angeordnet ist, wobei der Zufuhrschlauch (20b) dafür konfiguriert ist, Wasser von der Behandlungshaupteinheit (10) dem Behandlungshandstück (30) zuzuführen, und
einen Ausstoßschlauch (20c), der im Außenschlauch (20a) angeordnet und mit dem Zufuhrschlauch (20b) verbunden ist, wobei der Ausstoßschlauch (20c) dafür konfiguriert ist, Wasser von der Medienleitung (20) zum Behandlungshandstück (10) auszustoßen; und
eine Ventilanordnung, die dafür konfiguriert ist, das Wasser von der Behandlungshaupteinheit (10) entweder zu dem Zufuhrschlauch (20b) und dem Behandlungshandstück (30) oder zu dem Zufuhrschlauch (20b) und zurück zur Behandlungshaupteinheit (10) über den Ausstoßschlauch (20c) strömen zu lassen, wobei
die Behandlungshaupteinheit (10) einen Medienzufuhrteil (51) enthält, der dafür konfiguriert ist, das Wasser dem Zufuhrschlauch (20b) zuzuführen,
die Ventilanordnung umfasst
ein Auf/Zu-Ventil (40a) für eine Zufuhr, das in der Behandlungshaupteinheit (10) zwischen dem Medienzufuhrteil (51) und dem Zufuhrschlauch (20b) angeordnet ist, und
ein Auf/Zu-Ventil (40b) des Zufuhrschlauchs, das in einem Teil einer Verbindung der Medienleitung (20) mit dem Behandlungshandstück (30) und zwischen dem Behandlungshandstück (30) und einem Verbindungsteil des Zufuhrschlauchs (20b) angeordnet ist, mit dem der Ausstoßschlauch (20c) verbunden ist,
die medizinische Vorrichtung (100) dafür konfiguriert ist, das Wasser von der Behandlungshaupteinheit (10) zum Zufuhrschlauch (20b) und über den Ausstoßschlauch (20c) zurück zur Behandlungshaupteinheit (10) strömen zu lassen, indem das Auf/Zu-Ventil (40a) für eine Zufuhr in der Behandlungshaupteinheit (10) geöffnet und das Auf/Zu-Ventil (40b) des Zufuhrschlauchs geschlossen wird,
der Medienzufuhrteil (51) mit einer Leitungswasserquelle (50a) verbunden ist und der Zufuhrschlauch (20b) dafür konfiguriert ist, Leitungswasser von der Leitungswasserquelle (50a) zuzuführen.

2. Medizinische Vorrichtung (100) nach Anspruch 1, wobei
das Behandlungshandstück (30) eine Vielzahl von Handgeräten umfasst, und
die medizinische Vorrichtung (100) dafür konfiguriert ist, das Wasser vom Zufuhrschlauch (20b) von zumindest einem der Handgeräte über den Ausstoßschlauch (20c) zur Behandlungshaupteinheit (10) strömen zu lassen, indem ein Öffnen und Schließen des Auf/Zu-Ventils (40a) für eine Zufuhr und des Auf/Zu-Ventils (40b) des Zufuhrschlauchs gesteuert wird, das für jedes Handgerät vorgesehen ist.

3. Medizinische Vorrichtung (100) nach Anspruch 1 oder 2, wobei
das Auf/Zu-Ventil (40b) des Zufuhrschlauchs dafür konfiguriert ist, mittels Luft aktiviert zu werden.

4. Medizinische Vorrichtung (100) nach einem der Ansprüche 1 bis 3, wobei
das Auf/Zu-Ventil (40a) für eine Zufuhr und das Auf/Zu-Ventil (40b) des Zufuhrschlauchs dafür konfiguriert sind, in Abstimmung mit einer Aktivierung eines Hauptschalters (166) der Behandlungshaupteinheit (10) aktiviert zu werden.

5. Medizinische Vorrichtung (100) nach einem der Ansprüche 1 bis 4, wobei die medizinische Vorrichtung (100) ferner einen Detektor (170) aufweist, um eine Qualität des Wassers zu überwachen, das in der Behandlungshaupteinheit (10) und/oder dem Zufuhrschlauch (20b) zurückbleibt, wobei
das Auf/Zu-Ventil (40a) für eine Zufuhr und das Auf/Zu-Ventil (40b) des Zufuhrschlauchs dafür konfiguriert sind, als Antwort auf eine Ausgabe vom Detektor (170) automatisch aktiviert zu werden.

6. Medizinische Vorrichtung (100) nach einem der Ansprüche 1 bis 5, wobei
der Zufuhrschlauch (20b) ein Wassereinspeisungsschlauch ist, durch den das Wasser von der Behandlungshaupteinheit (10) dem Behandlungshandstück (30) zugeführt wird.

7. Medizinische Vorrichtung (100) nach einem der Ansprüche 1 bis 6, wobei
der Ausstoßschlauch (20c) ein Ablaufschlauch ist, durch den das Wasser von der Medienleitung (20) zur Behandlungshaupteinheit (10) ausgestoßen wird.

8. Medizinische Vorrichtung (100) nach einem der Ansprüche 1 bis 7, wobei
die Behandlungshaupteinheit (10) umfasst
einen Reinigungstank (130), der das aus dem Ausstoßschlauch (20c) ausgestoßene Wasser speichert, und
einen Unterdrucktank (150), der mit dem Reinigungstank (130) verbunden ist,
die medizinische Vorrichtung (100) dafür konfiguriert ist, das im Reinigungstank (130) gespeicherte Wasser zum Unterdrucktank (150) strömen zu lassen.

## Revendications

1. Dispositif médical (100) comprenant :
une unité principale de traitement (10) ;
un conduit pour milieu (20) flexible connecté à l'unité principale de traitement (10) ;
une pièce à main de traitement (30) connectée de façon amovible au conduit pour milieu (20) et connectée de façon amovible à l'unité principale de traitement (10), le conduit pour milieu (20) étant interposé,
le conduit pour milieu (20) incluant
un tube externe (20a),
un tube d'alimentation (20b) agencé dans le tube externe (20a), le tube d'alimentation (20b) étant configuré pour amener de l'eau depuis l'unité principale de traitement (10) vers la pièce à main de traitement (30), et
un tube d'émission (20c) agencé dans le tube externe (20a) et connecté au tube d'alimentation (20b), le tube d'émission (20c) étant configuré pour émettre de l'eau depuis le conduit pour milieu (20) vers l'unité principale de traitement (10) ; et
un agencement de vanne configuré pour permettre à l'eau de s'écouler depuis l'unité principale de traitement (10) soit vers le tube d'alimentation (20b) et la pièce à main de traitement (30), soit vers le tube d'alimentation (20b) et de nouveau vers l'unité principale de traitement (10) par l'intermédiaire du tube d'émission (20c), dans lequel
l'unité principale de traitement (10) inclut une partie d'alimentation en milieu (51) configurée pour amener l'eau vers le tube d'alimentation (20b),
l'agencement de vanne inclut
une vanne de marche/arrêt d'alimentation (40a) agencée dans l'unité principale de traitement (10) entre la partie d'alimentation en milieu (51) et le tube d'alimentation (20b), et
une vanne de marche/arrêt du tube d'alimentation (40b) agencée dans une partie de connexion du conduit pour milieu (20) vers la pièce à main de traitement (30) et entre la pièce à main de traitement (30) et une partie de connexion du tube d'alimentation (20b) auquel le tube d'émission (20c) est connecté,
le dispositif médical (100) est configuré pour permettre à l'eau de s'écouler depuis l'unité principale de traitement (10) vers le tube d'alimentation (20b) et par l'intermédiaire du tube d'émission (20c) de nouveau vers l'unité principale de traitement (10) en ouvrant la vanne de marche/arrêt d'alimentation (40a) dans l'unité principale de traitement (10) et en fermant la vanne de marche/arrêt du tube d'alimentation (40b),
la partie d'alimentation en milieu (51) est connectée à une source d'eau du robinet (50a) et le tube d'alimentation (20b) est configuré pour amener de l'eau du robinet depuis la source d'eau du robinet (50a) .

2. Dispositif médical (100) selon la revendication 1, dans lequel
la pièce à main de traitement (30) inclut une pluralité de pièces à main, et
le dispositif médical (100) est configuré pour permettre à l'eau de s'écouler depuis le tube d'alimentation (20b) d'au moins l'une des pièces à main par l'intermédiaire du tube d'émission (20c) vers l'unité principale de traitement (10) en commandant l'ouverture et la fermeture de la vanne de marche/arrêt d'alimentation (40a) et de la vanne de marche/arrêt du tube d'alimentation (40b) fournies pour chaque pièce à main.

3. Dispositif médical (100) selon la revendication 1 ou 2, dans lequel
la vanne de marche/arrêt du tube d'alimentation (40b) est configurée pour être activée par air.

4. Dispositif médical (100) selon l'une des revendications 1 à 3, dans lequel
la vanne de marche/arrêt d'alimentation (40a) et la vanne de marche/arrêt du tube d'alimentation (40b) sont configurées pour être activées en coordination avec l'activation d'un commutateur principal (166) de l'unité principale de traitement (10).

5. Dispositif médical (100) selon l'une des revendications 1 à 4, le dispositif médical (100) comprenant en outre un détecteur (170) pour surveiller la qualité de l'eau qui reste dans au moins l'un de l'unité principale de traitement (10) et du tube d'alimentation (20b), dans lequel
la vanne de marche/arrêt d'alimentation (40a) et la vanne de marche/arrêt du tube d'alimentation (40b) sont configurées pour être automatiquement activées en réponse à une sortie du détecteur (170).

6. Dispositif médical (100) selon l'une des revendications 1 à 5, dans lequel
le tube d'alimentation (20b) est un tube d'alimentation en eau à travers lequel l'eau est acheminée depuis l'unité principale de traitement (10) vers la pièce à main de traitement (30).

7. Dispositif médical (100) selon l'une des revendications 1 à 6, dans lequel
le tube d'émission (20c) est un tube de vidange à travers lequel l'eau est émise depuis le conduit pour milieu (20) vers l'unité principale de traitement (10).

8. Dispositif médical (100) selon l'une des revendications 1 à 7, dans lequel
l'unité principale de traitement (10) inclut
une cuve de nettoyage (130) qui stocke l'eau émise depuis le tube d'émission (20c), et
une cuve à vide (150) connectée à la cuve de nettoyage (130),
le dispositif médical (100) est configuré pour permettre à l'eau stockée dans la cuve de nettoyage (130) de s'écouler dans la cuve à vide (150).
